(19) **Europäisches Patentamt / European Patent Office / Office européen des brevets**

(11) **EP 4 377 692 B1**

(12) # EUROPEAN PATENT SPECIFICATION

(45) Date of publication and mention of the grant of the patent:
**03.09.2025  Bulletin 2025/36**

(21) Application number: **21777360.5**

(22) Date of filing: **28.07.2021**

(51) International Patent Classification (IPC):
**G01N 33/28** (2006.01)  **G01N 29/02** (2006.01)
**G01N 29/036** (2006.01)

(52) Cooperative Patent Classification (CPC):
**G01N 33/2823; G01N 29/022; G01N 29/036; G01N 33/2835;** G01N 2291/0226

(86) International application number:
**PCT/IB2021/000539**

(87) International publication number:
**WO 2023/007205 (02.02.2023 Gazette 2023/05)**

(54) **METHOD OF ESTIMATING AN UNSTABLE ASPHALTENE CONTENT IN AN OIL SAMPLE**

VERFAHREN ZUR SCHÄTZUNG EINES INSTABILEN ASPHALTENGEHALTS IN EINER ÖLPROBE

PROCEDE D'ESTIMATION D'UNE TENEUR EN ASPHALTENE INSTABLE DANS UN ECHANTILLON DE PETROLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**05.06.2024  Bulletin 2024/23**

(73) Proprietors:
- **TotalEnergies OneTech**
  **92400 Courbevoie (FR)**
- **Centre National de la Recherche Scientifique (CNRS)**
  **75016 Paris (FR)**
- **UNIVERSITE DE PAU ET DES PAYS DE L'ADOUR**
  **64000 Pau (FR)**

(72) Inventors:
- **SAIDOUN, Mohamed**
  **64000 Pau Cedex (FR)**
- **DARIDON, Jean-Luc**
  **64013 Pau Cedex (FR)**
- **CARRIER, Hervé**
  **64013 Pau Cedex (FR)**

(74) Representative: **Plasseraud IP**
**104 Rue de Richelieu**
**CS92104**
**75080 Paris Cedex 02 (FR)**

(56) References cited:
**US-A- 6 087 662**

- **SAIDOUN MOHAMED: "Doctoral thesis - Investigations into Asphaltenes Destabilization, Aggregation and Deposition", 26 March 2020 (2020-03-26), pages 1 - 240, XP055910131, Retrieved from the Internet <URL:https://tel. archives-ouvertes.fr/tel-03459429/document> [retrieved on 20220413]**
- **GMACHOWSKI LECH ET AL: "Modeling of asphaltene aggregates structure and deposition", COLLOIDS AND SURFACES A: PHYSIOCHEMICAL AND ENGINEERING ASPECTS, ELSEVIER, AMSTERDAM, NL, vol. 484, 18 August 2015 (2015-08-18), pages 402 - 407, XP029271995, ISSN: 0927-7757, DOI: 10.1016/J.COLSURFA.2015.08.021**
- **ABDOLRAZZAGHI MOHAMMAD ET AL: "Dual Active Resonator for Dispersion Coefficient Measurement of Asphaltene Nano-Particles", IEEE SENSORS JOURNAL, IEEE, USA, vol. 17, no. 22, 15 November 2017 (2017-11-15), pages 7248 - 7256, XP011672281, ISSN: 1530-437X, [retrieved on 20171023], DOI: 10.1109/ JSEN.2017.2734692**

# EP 4 377 692 B1

## Description

### Technical Field

This disclosure pertains to the petrochemical field and more particularly to a method for estimating an unstable asphaltene content in an oil sample.

Such method may be used to determine a solubility curve of asphaltenes in the oil sample.

### Background Art

Asphaltenes are known to cause problems in oil production conduits by destabilizing and causing operational difficulties.

The more oil reaches the surface, the more the pressure decreases. Under such conditions, the volume fraction of light constituents present in the oil increases and the density of the carrier liquid decreases at pressures larger than the saturation point. This leads to destabilization of asphaltenes present in the oil and to the formation of deposits. Thus, these asphaltenes tend to settle or to accumulate on the inner walls of the pipes, reducing their inner diameter and disturbing the flow of the fluid. Asphaltene destabilization is a thermodynamically reversible phenomenon, they tend to dissolve back into the oil when light constituents leave the liquid phase while forming a gas phase in pipelines.

Currently, laboratory tests are being performed under pressure on samples for which there is a risk of solid layer formation on the inner wall of the pipes. These tests aim to determine an "asphaltene onset pressure" parameter commonly referred to as AOP, i.e. the pressure below which micrometer sized aggregated solid asphaltene particles can be observed flowing in the oil. It is assumed that this pressure corresponds to the appearance of problems related to the accumulation of asphaltenes on the walls. These methods tend to localize the place in the pipe network where asphaltene deposits can occur. Once the key locations where asphaltene deposits tend to occur are determined, chemicals are injected in order to limit the formation of deposits and solvents are regularly used to clean up the pipes while the oil extraction is temporarily stopped.

However, there is no quantitative and accurate way to say how much deposit mass will be formed per unit time during operations and to estimate the frequency of cleaning jobs and associated production shortfalls.

In order to obtain the thickness of the asphaltene deposit under real conditions, i.e. in the pipes, it is useful to determine a concentration parameter of asphaltenes that are unstable in the sense that they tend to agglomerate and deposit on surfaces such as pipe walls.

Currently, concentration curves are obtained by measuring a solid formation concentration under atmospheric pressure conditions and using physical separation methods, such as centrifuges or filters. In one of the methods, called centrifugation method, asphaltenes are destabilized by mixing them with a liquid alkane at atmospheric pressure. Indeed, each alkane has a different effect on the nature and the number of destabilized asphaltenes. Currently, thermodynamic prediction calculation may be used to extrapolate results to field conditions of pressure and temperature.

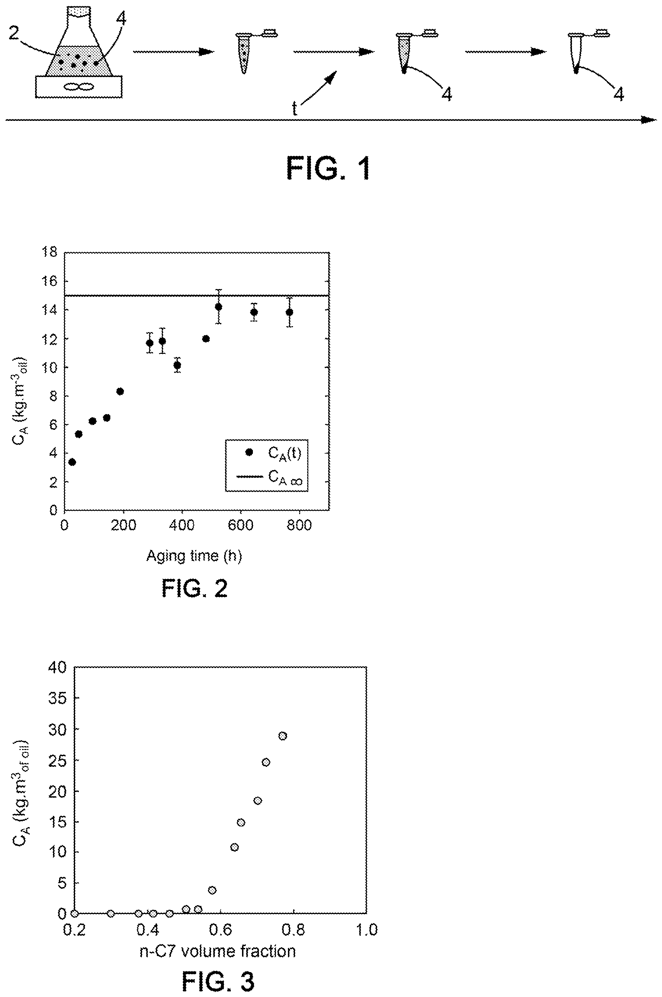

The centrifugation method is illustrated in Figure 1. A liquid alkane is added to crude oil and stirred. A solid phase 4 is then formed in the mixture 2. A quantity of liquid with dispersed solid asphaltenes is then removed after centrifugation. The solid asphaltenes are cleaned and a mass of an asphaltene pellet is obtained. As illustrated in figure 2, by dividing the mass of separated and washed asphaltenes by the amount of oil present at the beginning of the experiment, a graph of unstable asphaltene concentration $C_A$ versus aging time is obtained. The aging time represents the time of the experiment for each measurement. After a while, typically a few hundred hours, an equilibrium concentration, represented by a line, is reached.

As shown in Figure 3, this centrifugation method also provides a discrete plot of asphaltene solubility versus volumetric composition of the mixture. Specifically, this plot represents an unstable asphaltene concentration $C_A$ as a function of heptane volume fraction in the mixture of oil-heptane. Volumetric composition of the mixture can be varied by other means, i.e. varying the pressure or the temperature due to the compression or expansion of light constituents. By this method, points of unstable asphaltene concentration are obtained corresponding to discrete samples.

It is thus possible to make studies measuring the quantity of destabilized asphaltenes with liquid oil samples and alkanes at atmospheric pressure.

However, under real conditions, alkanes are dissolved gases such as methane, ethane or propane that will expand in the liquid when the pressure decreases while remaining higher than the saturation point. The relevant variable to the solubility of asphaltenes remains the volumetric composition of the liquid. But for practical reasons, in the laboratory, physical separation methods, such as the centrifugation method, were developed at atmospheric pressure with alkanes that are liquid at this atmospheric pressure with significant volume of samples. Separation methods applied at atmospheric pressure are therefore not representative of real conditions, i.e. conditions of oil extraction. Separation methods are complicated to implement on large enough quantities of discrete pressurized samples. In addition, visual observations cannot be made on opaque pressurized containers to verify that asphaltenes do not undergo a reversible stabilization when decreasing the pressure to recover and weight solid pellets.

Moreover, phenomena of asphaltene destabilization and deposition are continuous. Indeed, in laboratory conditions, a certain quantity of alkane needs to be added and then the mixture must rest to reach a measurable quantity using a weighting scale, as opposed to aging occurring simultaneously with depressurization in real conditions. In laboratory conditions, the used alkane is continuously present after its addition to the crude oil, a variable time and a variable volume fraction of alkane act in an interdependent way continuously. Thus, the existing laboratory methods are useful to study the kinetics for fixed concentrations of alkanes. However, they are not representative of what happens under real conditions where the volume fraction of compressible alkanes continuously changes in a part of flowing liquid.

In addition, using physical separation methods requires waiting for a long equilibration time (weeks). It is also necessary to obtain a sufficient quantity of precipitate to have an accurate measurement of the mass for each point.

Physical separation methods only allow to study aggregates which reached a minimum size enabling such separation (e.g., >0.2 micrometer) while the destabilization phenomenon is known to occur at a different length scale (e.g., <0.1 micrometer). FR 2 991 773 A1 discloses using a quartz crystal resonator (QCR) as a mass sensor in the determination of a pressure threshold above which flocculation of asphaltenes occurs in oil with a sensitivity to solid onset at the relevant length scale (< 0.01 micrometer). US 6087662A discloses a method for direct asphaltene concentration measurement by using IR spectroscopy.

## Summary

This disclosure proposes a method for estimating an unstable asphaltene content in an oil sample, the method comprising:

    a) placing a sensing device in a cell;
    b) causing the oil sample to flow in the cell;
    c) varying at a least one parameter among temperature of the oil sample, pressure and amount of alkanes added to the oil sample;
    d) measuring a deposition rate of asphaltenes deposited on the sensing device while the at a least one parameter varies; and
    e) calculating a concentration of unstable asphaltenes from the measured deposition rate of asphaltenes and a diffusion coefficient of primary unstable asphaltenes present in the oil sample, estimated based on a hydrodynamic radius of the primary unstable asphaltenes.

The present method enables determining the total concentration of unstable asphaltenes by taking into account a diffusion coefficient of primary unstable asphaltenes. The "primary" unstable asphaltenes refer to the asphaltene components that first lose their stability and form aggregates with other asphaltene components, which aggregates may grow further and/or settle on pipe walls. The primary unstable asphaltenes are considered as the predominant contributors to deposition on contacted solid surfaces under flowing conditions, as a function of time. This method allows to estimate the concentration of unstable asphaltenes or solubility profile of asphaltenes of crude oil by performing a continuous and single experiment requiring limited volumes of samples. The method applies to investigate the effect of pressure, temperature and any alkane addition on asphaltenes solubility curve. The rate of asphaltenes deposition in oil conduits is subsequently calculated and consequently, in operations, the frequency of cleaning jobs needed to eliminate the asphaltenes deposit for improving the oil recovery can be optimally determined.

The hydrodynamic radius $R_A$ of the primary unstable asphaltenes may be between 7 and 10 nm.

A single hydrodynamic radius is preferably used in this method and is representative of the whole process of asphaltenes deposition. It contributes to making the method analytically applicable.

The diffusion coefficient $D_A$ of unstable asphaltenes may be estimated as:

$$D_A = \frac{k_B T}{6\pi\mu R_A}$$

where:

-   $k_B$ is Boltzmann's constant;
-   T is the temperature of the oil sample;
-   $\mu$ is the dynamic viscosity of the oil sample; and
-   $R_A$ is the hydrodynamic radius of the primary unstable asphaltenes.

In an embodiment, the concentration of unstable asphaltenes is calculated as being proportional to $D_A^{-2/3}$.

The concentration $C_A$ of unstable asphaltenes may be calculated as a sum of concentrations of the primary unstable asphaltenes over consecutive time intervals.

The concentration of the primary unstable asphaltenes over an $i^{th}$ time interval may be proportional to the measured rate of deposition of asphaltenes on the sensing device during the $i^{th}$ time interval, $Rate_{deposition_i}$. In an example, it is taken as $\dfrac{Rate_{deposition_i}}{0.664 a_c \; D_A^{2/3} \left(\dfrac{U_0}{L}\right)^{1/2} \left(\dfrac{\rho}{\mu}\right)^{1/6}}$ , where:

- $a_c$ is a surface area of the sensing device where deposition occurs;
- $U_0$ is the superficial velocity of the oil sample in the cell at the sensing device;
- L is a characteristic length of a geometry where the oil sample flows;
- $\rho$ is the density of the oil sample;
- $\mu$ is the dynamic viscosity of the oil sample.

The concentration $C_A(t_f)$ of unstable asphaltenes at an experiment time $t_f$ is then obtained as:

$$C_A(t_f) \sim \sum_{i=1}^{n} \left( \frac{Rate_{deposition_i}}{0.664 a_c \; D_A^{2/3} \left(\frac{U_0}{L}\right)^{1/2} \left(\frac{\rho}{\mu}\right)^{1/6}} \right)$$

where n is a number of time intervals to reach $t_f$.

The sensing device may comprise a quartz crystal resonator.

A solubility curve of asphaltenes in the oil sample may be obtained based on the estimated unstable asphaltene content.

**Brief Description of the Drawings**

Fig. 1 is a schematic representation of steps of a centrifugation method;

Fig. 2 is a graph representing a concentration of asphaltenes versus aging time;

Fig. 3 is a graph representing a concentration of asphaltenes versus heptane volume fraction;

Fig. 4 is a graph representing an increment of a primary unstable asphaltene quantity per unit time and the rate of deposition as a function of heptane volume fraction during continuous addition of heptane;

Fig. 5 is a graph representing a mass of asphaltenes deposit versus heptane volume fraction;

Fig. 6 is a graph representing the mass of asphaltenes deposit versus methane volume fraction;

Fig. 7 is a graph representing a rate of deposition versus a pressure for three pressurization speeds;

Fig. 8 is a graph representing the rate of deposition versus methane volume fraction for the same three pressurization speeds as in figure 8;

Fig. 9 is a graph representing the concentration of unstable asphaltenes versus methane volume fraction.

Fig. 10 is a graph representing the concentration of unstable asphaltenes versus methane volume fraction according to the invention and a centrifugation method.

**Description of Embodiments**

The present document proposes a method for estimating an unstable asphaltene content in an oil sample for various conditions of the oil, including temperature, pressure and in presence of solvents such as liquid alkanes at atmospheric pressure and/or dissolved gaseous light constituents in oil under pressurized conditions. In the remainder of the present document, the expression "liquid alkanes" will be simplified by "alkanes".
From the estimation of unstable asphaltene content, a solubility curve of asphaltenes in the oil sample may be calculated upon the various thermodynamic and compositional conditions of the oil.

An a priori unknown parameter that governs the determination of the asphaltene deposit mass is a diffusion coefficient of unstable asphaltenes contributing to a deposition process.

In a pipeline, the shallower a fluid, the lower the pressure of the fluid due to hydrostatic and frictional pressure losses of the flowing fluid. Thus, compressible compounds contained in the oil expand upon flowing up and a phenomenon of asphaltenes destabilization occurs. Nanoaggregates of asphaltenes, originally dispersed and stable, with sizes between 7 and 10 nanometers, become unstable due to the lack of ability of the surrounding solvent to solubilize them. The solvent consists mostly of alkanes. When the solvent changes in volume composition, the asphaltenes are destabilized. During this destabilization step where asphaltenes go from a stable to an unstable state, the size distribution of the asphaltenes does not change significantly (sizes can be slightly reduced, however, due to shrinking of asphaltene molecules to expulse the trapped solvent in nanoaggregate structures).

The inventors' observations have led to determining that the asphaltenes that primarily become unstable are in a relatively narrow size distribution. Namely, their sizes correspond to a typical range of hydrodynamic radius $R_A$ of 7 to 10 nm. Once they become unstable, they will aggregate with other unstable asphaltene particles.

The identification of the relevant size of unstable asphaltenes with regards to the deposition process allows the selection of the right diffusion coefficient $D_A$ of unstable asphaltenes for understanding their hydrodynamic behavior, using the following formula:

$$D_A = \frac{k_B T}{6\pi\mu R_A}$$

where:

- $k_B$ is Boltzmanns' constant;
- T is the temperature of the oil;
- $\mu$ is the dynamic viscosity of the oil; and
- $R_A$ is the average hydrodynamic radius of the primary unstable asphaltenes.

Once the primary unstable asphaltenes are formed, an aggregation phenomenon occurs, reducing their contact with the surrounding fluid. Thus, the primary unstable asphaltenes grow from a size between 7 and 10 nanometers to microscopic sizes. These larger asphaltenes are transported with the flow and do not diffuse toward the inner wall.

If all unstable asphaltenes can deposit and diffuse to the wall, a variety of diffusion coefficients corresponding to a multitude of unstable asphaltene sizes would be obtained. Under such conditions, a bad estimate of the amount of deposition is gotten from this multitude of diffusion coefficients varying by a factor of 1000 to 10000. In previous practice, diffusion coefficients of the polydisperse unstable asphaltenes were usually obtained by first tuning an aggregation numerical model. However, recent findings show that destabilization and aggregation kinetics occur simultaneously, additional uncertainties related to the simulation of the aggregation process would complicate the calculation of a distribution of diffusion coefficients. In this method, only primary particles of unstable asphaltenes are assumed to contribute to the diffusive deposition process.

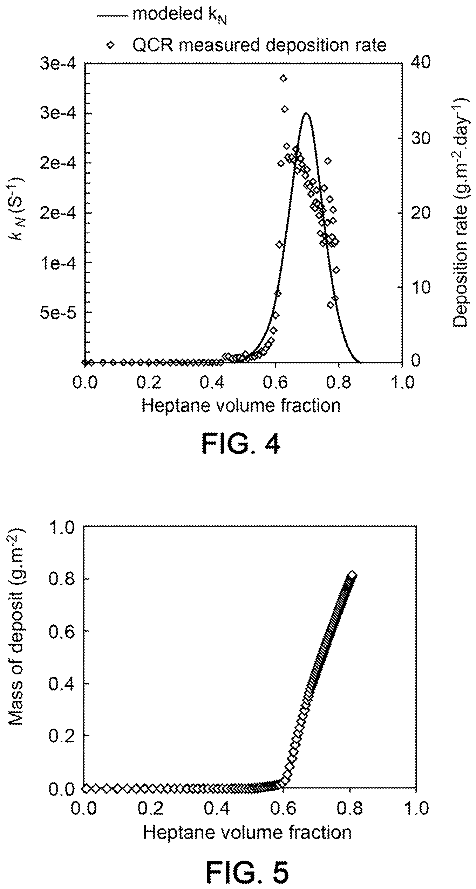

The graph in Figure 4 shows a destabilization coefficient on the ordinate representing an increment of a primary unstable asphaltene quantity per unit time along with the rate of deposition in $g.m^{-2}.day^{-1}$ as a function of a given heptane volume fraction during its continuous addition to oil. A good correlation of these two parameters, the increment of a primary unstable asphaltene quantity per unit time and the rate deposition, is observed which confirms that the asphaltenes contributing to the deposition are the primary unstable asphaltenes.

The present method is applied using a sensing device configured to measure the mass of asphaltene deposits on a known surface area. The sensing device may be a quartz crystal resonator (QCR) immersed in an oil sample. The QCR may be installed in a temperature and pressure-controlled piston cell in which the oil sample flows. Fluid inlets and outlets respectively toward and from the cell are controlled using pumps and overflow valves. The mechanical stirring rate is also controlled to evaluate the average superficial velocity $U_0$ of the oil sample in the vicinity of the quartz crystal installed in the cell.

Classically, the QCR resonates at a characteristic resonance frequency varying according to the fluid in which it is immersed. This resonance frequency also varies in the presence of solids formed on its surface. The signal from the quartz crystal is interpreted in a known manner to give access to a deposit mass of asphaltenes formed on the QCR. The way this equipment works is described in the document FR 2 991 773 A1.

From the resonance frequency of the quartz crystal at multiple frequencies, a measurement of the deposit mass on the quartz crystal is obtained.

As illustrated in figure 5, a follow-up of the deposit mass on the quartz crystal in unit of mass per unit area [g.m$^{-2}$] over time is obtained, during the continuous addition of alkane. In the graph, the alkane used is heptane, but the present invention is not limited to this alkane. Various alkanes can be used, including compressible constituents.

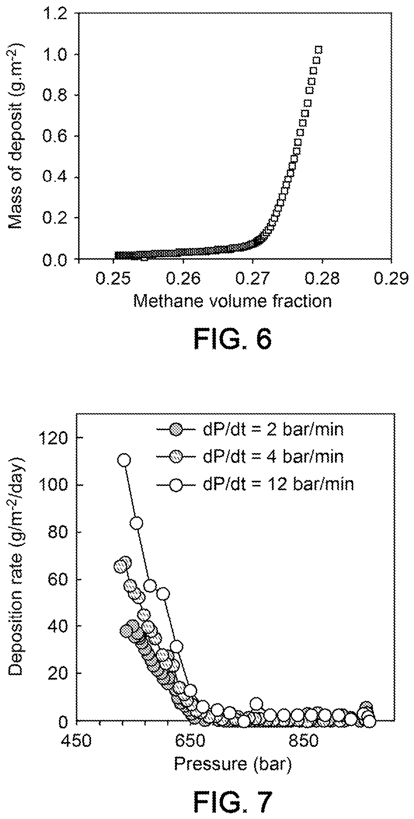

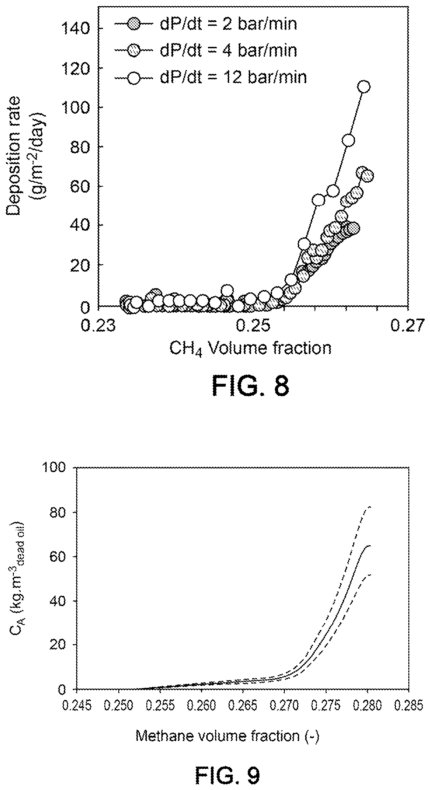

When depressurizing a fluid with dissolved gases, the same type of graph is obtained, as shown in figure 6. More details are given for dissolved gases in figure 7 and figure 8. Figure 7 represents the rate of deposition as a function of pressure for three different pressurization speeds: 2 bar/min; 4 bar/min and 12 bar/min. Figure 8 represents the same rate of deposition as in figure 7 but as a function of methane volume fraction for the same three depressurization speeds. In the present example, the alkane used is methane. The curves obtained are continuous and pressurized conditions unlike the prior art.

From the measured mass of asphaltenes deposited on the QCR, the rate of deposition can be obtained by dividing each incremental measured mass by the incremental time. A calculation of a total concentration $C_A$ of unstable asphaltenes at an experiment time $t_f$ is obtained from a Riemann-type sum:

$$C_A(t_f) \sim \sum_{i=1}^{n} \left( \frac{Rate_{deposition_i}}{0.664 a_c \ D_A{}^{2/3} \left(\frac{U_0}{L}\right)^{1/2} \left(\frac{\rho}{\mu}\right)^{1/6}} \right)$$

where:

- n is a number of time intervals to reach $t_f$;

- $Rate_{deposition_i}$ is a measured mass rate of deposition of asphaltenes on the sensing device during the i$^{th}$ time interval, i.e. $Rate_{deposition;i} = \Delta m_i / \Delta t_i$, where $\Delta m_i$ is a mass increment of the deposited asphaltenes during the i$^{th}$ time interval of a short duration equal to $\Delta t_i$, where $1 \leq i \leq n$;

- $D_A$ is the estimated diffusion coefficient of the primary unstable asphaltenes;

- $a_c$ is the surface area of the sensing device where deposition occurs;

- $U_0$ is the superficial velocity of the oil sample in the cell at the sensing device;

- L is a characteristic length of a geometry where the oil sample flows;

- $\rho$ is the density of the oil sample;

- $\mu$ is the dynamic viscosity of the oil sample.

In an embodiment, the temperature and pressure are fixed and along the experiment, the quantity of solvent is increased from 0% up to 99%, more preferably from 0 up to 80%. Then, the values of temperature and pressure are changed and fixed. The quantity of solvent is again increased from 0% up to 99%, more preferably from 0% up to 80%. At each variation of the quantity of solvent, the deposition rate is measured using the QCR and the total concentration $C_A$ of unstable asphaltenes is calculated.

As illustrated in figure 9, a graph of the total concentration $C_A$ of unstable asphaltenes in kg.m$^{-3}$ of a dead oil is obtained as a function of the volume fraction of alkane, such as methane or heptane by applying the above equation. The dead oil refers to degassed liquid oil at atmospheric standard conditions. It is possible to vary experimental conditions such as pressure, temperature and molar composition of the liquid.

The parameter $C_A$ representing the total concentration of unstable asphaltenes can be entered into simulators under real flow conditions. A thickness and/or mass of the asphaltene deposit forming in a pipe can thus be estimated.

The following equation gives the deposition rate $Rate_{deposition}$ in a pipe including a characteristic cross section area parameter of the pipe $a_p$, a characteristic diameter d of the pipe, the estimated diffusion coefficient of primary unstable asphaltenes $D_A$, a fluid superficial velocity $U_0$, a density $\rho$ and a viscosity $\mu$ of the fluid at the relevant conditions:

$$Rate_{deposition} \sim 0.026 a_p D_A^{\frac{2}{3}} U_0^{\frac{4}{5}} d^{-\frac{1}{5}} \left(\frac{\rho}{\mu}\right)^{3/5} \Delta C_A$$

The rate of deposition Rate$_{deposition}$ is obtained from the concentration of primary unstable asphaltenes, assumed to correspond to the increment $\Delta C_A$ across a change of liquid solution and time.

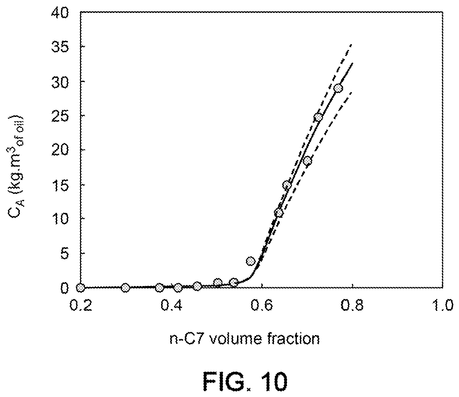

In case it is possible to work at atmospheric pressure, a comparison between the curve obtained by this method using a QCR and the curve obtained with the centrifugation method showed an excellent correlation as illustrated in figure 10. Indeed, figure 10 represents in full line the total concentration $C_A$ of unstable asphaltenes as a function of heptane volume fraction in a mixture of oil and heptane, calculated for a hydrodynamic radius $R_A$ equal to 7nm. Figure 10 represents in dashed line the total concentration $C_A$ of unstable asphaltenes as a function of heptane volume fraction in a mixture of oil and heptane, calculated for a hydrodynamic radius $R_A$ equal to 8.4 nm and 5.6 nm. Finally, the dots are the results obtained by the conventional centrifugation method at atmospheric pressure. The results obtained by both methods match very well, while the method proposed herein is of a simpler implementation.

The method provides a continuous recording of cumulative unstable asphaltenes concentration during variations in pressure, temperature or alkane solvent addition.

This method is fast: a single test, and the equation leads to the solubility curve as a function of the surrounding solvent change in volumetric composition (caused by additional solvent, by temperature ramp or by depressurization of the liquid containing compressible elements).

This method allows to differentiate the asphaltenes responsible for the deposition from the whole destabilized asphaltenes. And, contrary to other weighting methods, it does not require large quantities of destabilized asphaltenes. The sensitivity of the measurement method is in the range of tens of nanograms.

Lastly, it is a non-destructive method on pressurized samples. Several tests are possible under different conditions and the sample can be reused for other purposes.

It will be appreciated that the embodiments described above are illustrative of the invention disclosed herein and that various modifications can be made without departing from the scope as defined in the appended claims.

**Claims**

1. Method for estimating an unstable asphaltene content in an oil sample, the method comprising:

   a) placing a sensing device in a cell;
   b) causing the oil sample to flow in the cell;
   c) varying at a least one parameter among temperature of the oil sample, pressure and an amount of alkanes added to the oil sample;
   d) measuring a deposition rate of asphaltenes deposited on the sensing device while the at a least one parameter varies; and
   e) calculating a concentration ($C_A$) of unstable asphaltenes from the measured deposition rate of asphaltenes and a diffusion coefficient ($D_A$) of primary unstable asphaltenes present in the oil sample, estimated based on a hydrodynamic radius ($R_A$) of the primary unstable asphaltenes.

2. Method according to claim 1, wherein the hydrodynamic radius ($R_A$3. of the primary unstable asphaltenes is between 7 and 10 nm.

3. Method according to any one of the preceding claims, wherein the diffusion coefficient $D_A$ of the primary unstable asphaltenes is estimated as:

$$D_A = \frac{k_B T}{6 \pi \mu R_A}$$

where:

   - $k_B$ is Boltzmann's constant;
   - T is the temperature of the oil sample;

- $\mu$ is the dynamic viscosity of the oil sample; and
- $R_A$ is the hydrodynamic radius of the primary unstable asphaltenes.

**4.** Method according to any one of the preceding claims, wherein the concentration ($C_A$) of unstable asphaltenes is calculated as being proportional to $D_A^{-2/3}$, where $D_A$ is the diffusion coefficient of the primary unstable asphaltenes.

**5.** Method according to any one of the preceding claims, wherein the concentration ($C_A$) of unstable asphaltenes is calculated as a sum of concentrations of the primary unstable asphaltenes over consecutive time intervals.

**6.** Method according to any one of the preceding claims, wherein the concentration $C_A(t_f)$ of unstable asphaltenes at an experiment time $t_f$ is calculated as:

$$C_A(t_f) \sim \sum_{i=1}^{n} \left( \frac{Rate_{deposition_i}}{0.664 a_c \; D_A^{2/3} \left( \frac{U_0}{L} \right)^{1/2} \left( \frac{\rho}{\mu} \right)^{1/6}} \right)$$

where:

- n is a number of time intervals to reach $t_f$;
- $Rate_{deposition_i}$ is a measured rate of deposition of asphaltenes on the sensing device during the ith time interval ;
- $D_A$ is the estimated diffusion coefficient of the primary unstable asphaltenes;
- $a_c$ is a surface area of the sensing device where deposition occurs;
- $U_0$ is the superficial velocity of the oil sample in the cell at the sensing device;
- L is a characteristic length of a geometry where the oil sample flows;
- $\rho$ is the density of the oil sample;
- $\mu$ is the dynamic viscosity of the oil sample.

**7.** Method according to any one of the preceding claims, wherein the sensing device comprises a quartz crystal resonator.

**8.** Method according to any one of the preceding claims, wherein a solubility curve of asphaltenes in the oil sample is obtained based on the estimated unstable asphaltene content.

**Patentansprüche**

**1.** Verfahren zum Schätzen eines Gehalts instabiler Asphaltene in einer Ölprobe, das Verfahren umfassend:

a) Platzieren einer Erfassungsvorrichtung in einer Zelle;
b) Veranlassen, dass die Ölprobe in die Zelle fließt;
c) Variieren von mindestens einem Parameter aus einer Temperatur der Ölprobe, einem Druck und einer Menge von Alkanen, die zu der Ölprobe hinzugefügt werden;
d) Messen einer Ablagerungsrate von Asphaltenen, die sich auf der Erfassungsvorrichtung abgelagert haben, während der mindestens eine Parameter variiert; und
e) Berechnen einer Konzentration ($C_A$) von instabilen Asphaltenen aus der gemessenen Ablagerungsrate von Asphaltenen und eines Diffusionskoeffizienten ($D_A$) von primären instabilen Asphaltenen, die in der Ölprobe vorliegen, geschätzt auf Basis eines hydrodynamischen Radius ($R_A$) der primären instabilen Asphaltene.

**2.** Verfahren nach Anspruch 1, wobei der hydrodynamische Radius ($R_A$) der primären instabilen Asphaltene zwischen 7 und 10 nm beträgt.

**3.** Verfahren nach einem der vorhergehenden Ansprüche, wobei der Diffusionskoeffizient $D_A$ der primären instabilen Asphaltene geschätzt wird als:

$$D_A = \frac{k_B T}{6\pi\mu R_A}$$

wobei:

- $k_B$ die Boltzmann-Konstante ist;
- T die Temperatur der Ölprobe ist;
- $\mu$ die dynamische Viskosität der Ölprobe ist; und
- $R_A$ der hydrodynamische Radius der primären instabilen Asphaltene ist.

4. Verfahren nach einem der vorhergehenden Ansprüche, wobei die Konzentration ($C_A$) von instabilen Asphaltenen als proportional zu $D_A^{-2/3}$, berechnet wird, wobei $D_A$ der Diffusionskoeffizient der primären instabilen Asphaltene ist.

5. Verfahren nach einem der vorhergehenden Ansprüche, wobei die Konzentration ($C_A$) von instabilen Asphaltenen als eine Summe von Konzentrationen der primären instabilen Asphaltenen über aufeinanderfolgende Zeitintervalle berechnet wird.

6. Verfahren nach einem der vorhergehenden Ansprüche, wobei die Konzentration $C_A(t_f)$ von instabilen Asphaltenen bei einer Experimentzeit $t_f$ berechnet wird als:

$$C_A(t_f) \sim \sum_{i=1}^{n} \left( \frac{Rate_{deposition_i}}{0.644 a_c \, D_A^{2/3} \left(\frac{U_0}{L}\right)^{1/2} \left(\frac{\rho}{\mu}\right)^{1/6}} \right)$$

wobei:

- n eine Anzahl von Zeitintervallen ist, um $t_f$ zu erreichen;
- $Rate_{deposition_i}$ eine gemessene Rate einer Ablagerung von Asphaltenen auf der Erfassungsvorrichtung während des i-ten Zeitintervall ist;
- $D_A$ der geschätzte Diffusionskoeffizient der primären instabilen Asphaltene ist;
- $a_c$ ein Flächenbereich der Erfassungsvorrichtung ist, an dem eine Ablagerung auftritt;
- $U_0$ die Oberflächengeschwindigkeit der Ölprobe in der Zelle an der Erfassungsvorrichtung ist;
- L eine charakteristische Länge einer Geometrie ist, an der die Ölprobe fließt;
- $\rho$ die Dichte der Ölprobe ist;
- $\mu$ die dynamische Viskosität der Ölprobe ist.

7. Verfahren nach einem der vorhergehenden Ansprüche, wobei die Erfassungsvorrichtung einen Quarzkristallresonator umfasst.

8. Verfahren nach einem der vorhergehenden Ansprüche, wobei eine Löslichkeitskurve von Asphaltenen in der Ölprobe auf Basis des geschätzten Gehalts instabiler Asphaltene erhalten wird.

**Revendications**

1. Procédé d'estimation d'une teneur en asphaltènes instables dans un échantillon d'huile, le procédé comprenant :

a) la mise en place d'un dispositif de détection dans une cellule ;
b) la mise en écoulement de l'échantillon d'huile dans la cellule ;
c) la variation d'au moins un paramètre parmi la température de l'échantillon d'huile, la pression et une quantité d'alcanes ajoutée à l'échantillon d'huile ;
d) la mesure d'un taux de dépôt d'asphaltènes déposés sur le dispositif de détection pendant que ledit au moins un paramètre varie ; et
e) le calcul d'une concentration ($C_A$) d'asphaltènes instables à partir du taux de dépôt mesuré d'asphaltènes et d'un coefficient de diffusion (D$_A$) d'asphaltènes instables primaires présents dans l'échantillon d'huile, estimé sur la base d'un rayon hydrodynamique (R$_A$) des asphaltènes instables primaires.

**2.** Procédé selon la revendication 1, dans lequel le rayon hydrodynamique (R$_A$) des asphaltènes instables primaires est compris entre 7 et 10 nm.

**3.** Procédé selon l'une quelconque des revendications précédentes, dans lequel le coefficient de diffusion D$_A$ des asphaltènes instables primaires est estimé comme suit :

$$D_A = \frac{k_B T}{6\pi\mu R_A}$$

où :

- k$_B$ est la constante de Boltzmann ;
- T est la température de l'échantillon d'huile ;
- $\mu$ est la viscosité dynamique de l'échantillon d'huile ; et
- R$_A$ est le rayon hydrodynamique des asphaltènes instables primaires.

**4.** Procédé selon l'une quelconque des revendications précédentes, dans lequel la concentration (C$_A$) d'asphaltènes instables est calculée comme étant proportionnelle à $\boldsymbol{D_A^{-2/3}}$, où D$_A$ est le coefficient de diffusion des asphaltènes instables primaires.

**5.** Procédé selon l'une quelconque des revendications précédentes, dans lequel la concentration (C$_A$) d'asphaltènes instables est calculée comme étant une somme de concentrations des asphaltènes instables primaires sur des intervalles de temps consécutifs.

**6.** Procédé selon l'une quelconque des revendications précédentes, dans lequel la concentration C$_A$(t$_f$) d'asphaltènes instables à un instant expérimental t$_f$ est calculée comme suit :

$$C_A(t_f) \sim \sum_{i=1}^{n} \left( \frac{Rate_{deposition_i}}{0.644 a_c \, D_A^{2/3} \left(\frac{U_0}{L}\right)^{1/2} \left(\frac{\rho}{\mu}\right)^{1/6}} \right)$$

où :

- n est le nombre d'intervalles de temps pour atteindre t$_f$ ;
- $\boldsymbol{Rate_{deposition_i}}$ est un taux de dépôt mesuré d'asphaltènes sur le dispositif de détection pendant le i$^e$ intervalle de temps ;
- D$_A$ est le coefficient de diffusion estimé des asphaltènes instables primaires ;
- a$_c$ est une zone de surface du dispositif de détection sur laquelle le dépôt a lieu ;
- U$_0$ est la vitesse superficielle de l'échantillon d'huile dans la cellule au niveau du dispositif de détection ;
- L est une longueur caractéristique d'une géométrie où s'écoule l'échantillon d'huile ;
- $\rho$ est la densité de l'échantillon d'huile ;
- $\mu$ est la viscosité dynamique de l'échantillon d'huile.

**7.** Procédé selon l'une quelconque des revendications précédentes, dans lequel le dispositif de détection comprend un résonateur à cristal de quartz.

**8.** Procédé selon l'une quelconque des revendications précédentes, dans lequel une courbe de solubilité d'asphaltènes dans l'échantillon d'huile est obtenue sur la base de la teneur estimée en asphaltènes instables.

FIG. 1

FIG. 2

FIG. 3

FIG. 4

FIG. 5

**FIG. 6**

**FIG. 7**

FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2991773 A1
- US 6087662 A